# EUROPEAN PATENT SPECIFICATION

(11) **EP 1 333 841 B1**
(45) Date of publication and mention of the grant of the patent: **24.05.2006**
(21) Application number: 01983701.2
(22) Date of filing: 16.11.2001
(51) Int. Cl.: A61K 31/711, A61K 39/04, A61P 31/04, G01N 33/68

(54) **M. TUBERCULOSIS CHAPERONIN 10 AND USES THEREOF**
M. TUBERCULOSIS CHAPERONIN 10 UND SEINE VERWENDUNGEN
M. TUBERCULOSIS CHAPERONIN 10 ET SES UTILISATIONS

(30) Priority: 17.11.2000 GB 0028122
(43) Date of publication of application: 13.08.2003
(73) Proprietor: Helperby Therapeutics Limited, Selby North Yorkshire YO8 4PW (GB)
(72) Inventor: COATES, Anthony Robert Milnes Dept. of Med. Mic., Tooting London SW17 0RE (GB)
(74) Representative: Thomas, Philip John Duval
(86) International application number: PCT/GB2001/005084
(87) International publication number: WO 2002/040038

(56) References cited:
- WO-A-01/04344
- BAIRD P N ET AL: "CLONING AND SEQUENCE ANALYSIS OF THE 10-KDA ANTIGEN GENE OF MYCOBACTERIUM-TUBERCULOSIS" JOURNAL OF GENERAL MICROBIOLOGY, vol. 135, no. 4, 1989, pages 931-940, XP008005161 ISSN: 0022-1287
- BAIRD P N ET AL: "A MAJOR ANTIGEN FROM MYCOBACTERIUM-TUBERCULOSIS WHICH IS HOMOLOGOUS TO THE HEAT SHOCK PROTEINS GRO-ES FROM ESCHERICHIA-COLI AND THE HTP-A GENE PRODUCT OF COXIELLA-BURNETII" NUCLEIC ACIDS RESEARCH, vol. 16, no. 18, 1988, page 9047 XP001074008 ISSN: 0305-1048
- JANEWAY, C., TRAVERS, P., HUNT, S., WALPORT, M.: "Immunobiology" 1997, CURRENT BIOLOGY AND GARLAND PUBLISHING , LONDON AND N. YORK

## Description

The present invention relates to the use of an approximately 10kDa polypeptide (or its encoding nucleic acid molecule) or functionally equivalent molecules or fragments thereof from *Mycobacterium tuberculosis* or related prokaryotes in the treatment of allergic airway diseases and respiratory diseases characterised by eosinophilic airway inflammation and airway hyper-responsiveness to methods of stimulating the production of immune response mediators, e.g. cytokines, *in vitro.*

Chaperonins are believed to stimulate the immune system at many levels simultaneously, including monocytes, macrophages, fibroblast-like cells, perhaps other types of cells, and T cells. The immune defences in mammals may be divided into the "innate" and "adaptive" defences. Those which are already in place, such as phagocytes, natural killer cells and complement are considered innate. On challenge, adaptive immunity is activated in the form of B and T lymphocytes. Chaperonins are known to act directly on the innate defence mechanisms, particularly on phagocytes. They also stimulate a powerful adaptive immune response, namely the production of antibody and the stimulation of T lymphocytes which in some cases may be protective. Notably they induce cytokine secretion which is thought to be important for host defences. In some cases however it is believed that the presence of chaperonins may be damaging to the host.

Ragno *et al* (1996) Clin Exp Immunol 103: 384-390 showed that an aqueous solution of a 10-kD heat shock protein (hsp10) from *Mycobacterium tuberculosis* delayed the onset and severity of adjuvant-induced arthritis (AA) in rats. AA is a model of autoimmune diseases, which are associated with an underlying over-reactivity of Th-1 cells. There is no suggestion that hsp10 could find utility in the treatment of allergic conditions such as asthma, rhinitis/hay fever, eczema, analphylaxis and the like, which, in contrast to autoimmune diseases (Th1 over-reacting) are associated with an underlying over-reactivity of Th-2 cells.

Chaperonins' role in autoimmune disease is controversial. Although infection/immunity with chaperonin-containing organisms is universal, and healthy people have T cell responses to self-chaperonins, including the production of chaperonin-specific antibodies, classical autoimmune disease is quite uncommon. So the presence of immune reactions to chaperonins may be incidental and unimportant.

The theory of molecular mimicry however suggests the involvement of chaperonins in autoimmune disease and is based on the high level of amino acid sequence conservation between chaperonins of microbial and mammalian origin. The theory proposes that during infection with a wide range of microbes, chaperonin epitopes that are shared between microbes and mammals stimulate T lymphocytes. According to this theory a high level of chaperonin presentation of shared chaperonin epitopes breaks tolerance to self-chaperonins and autoimmune disease develops.

Chaperonins obtained from tumours have been found to result in necrotic effects on those tumours. It is suggested that this may be achieved through enhancing immunological recognition of tumour antigens although the mechanism of this is not known. It therefore appears that chaperonins induce protective adaptive immunity against bacterial infection and cancer.

Allergic reactions, such as asthma, concern proportionally inappropriate or misdirected immune responses. The prevalence of asthma for example is increasing and effective therapies for treating all cases have not yet been found. Current treatment often uses immunosuppressive glucocorticosteroids, beta agonists, cromoglycate, leukotriene modifiers etc. which have numerous side-effects.

In such allergic reactions, high IgE levels occur and T helper lymphocyte-2 (Th2) immune responses predominate over Th1 responses resulting in an inflammatory response. Th1 responses are thought to be mainly protective against microbial infection and are promoted by cytokines, particularly interleukin-12 (IL12), IL-2 and interferon-γ. In contrast, Th2 responses, in the appropriate genetic background, are associated with harmful allergic tissue damage.

However, it has been suggested that in other conditions such as autoimmune disorders, e.g. adjuvant arthritis, overactive Th1 responses are causal of the disorder. Conversion of Th1 to Th2 or Th2 to Th1 responses may therefore have utility in treating the above described disorders.

Whilst it has been known that bacteria such as *L. moncytogenes, M. bovis and M. tuberculosis* can convert Th2 to Th1 responses, the molecules which is(are) responsible for this conversion have not been identified.

Suggestions in the art have however implicated a heat shock protein, hsp65, *from M leprae* which is able to induce Th1 responses (Lowrie et al., 1999, Nature, 400, p269-271; Bonato et al., 1998, Infect. Immun., 66, p169-175). The homologue, hsp65 from *M tuberculosis,* has the ability to stimulate human monocytes to synthesize pro-inflammatory cytokines and activate monocytes and human vascular endothelial cells (Friedland et al., 1993, Clin. Exp. Immunol., 91, p5862; Peetermans et al., 1995, Infect. Immun., 63, p3454-3458; Verdegaal, et al., 1996, J. Immunol., 157, p369-376).

Surprisingly it has now been found that another protein is able to affect the immunity of an individual and can be used for treating or preventing allergic airway disease and respiratory diseases characterized by eosinophilic airway inflammation and airway hyper-responsiveness.

This protein from *Mycobacterium tuberculosis* has surprisingly been found to have advantageous properties in treating the aforementioned conditions. This 10kDa molecule is termed *Mycobacterium tuberculosis* chaperonin 10 (Mtcpn 10).

Cpn 10 which is a chaperonin and a heat shock protein is under discrete transcriptional control to the molecules cpn 60.1 and 60.2.

The invention therefore provides molecules such as *Mycobacterium* cpn 10 which have enhanced properties in treating or preventing allergic airway disease and respiratory diseases characterized by eosinophilic airway inflammation and airway hyper-responsiveness. Therapeutic and/or prophylactic applications may be achieved using nucleic acid molecules or peptides/proteins, as will be described in more detail hereinafter.

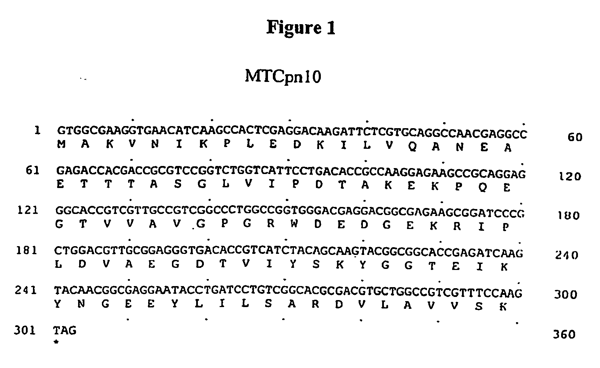

Thus, in a first aspect the present invention provides the use of a pharmaceutical composition comprising a nucleic acid molecule comprising
(i) the nucleotide sequence of Figure 1, or
(ii) a sequence which has more than 66%, e.g. 70 or
75%, preferably more than 80%, e.g. more than 90 or 950% identity to sequence (i) (according to the test described hereinafter) or a sequence which hybridizes to sequence (i) under conditions of 2 x SSC, 65°C (wherein SCC = 0.15M NaCl, 0.015M sodium citrate, pH 7.2) which encodes a functionally equivalent protein to the sequence encoded by the nucleotide sequence of Figure 1, or (iii) a fragment of any sequence (i) or (ii) encoding a functionally equivalent protein fragment; and a pharmaceutically acceptable excipient, diluent or carrier.

For the manufacture of a medicament for the prevention and/or treatment of allergic airway disease and respiratory diseases characterised by eosinophilic airway inflammation and airway hyper-responsiveness.

As mentioned above, therapeutic and/or prophylactic effects may be achieved using nucleic acid molecules or peptide/protein molecules. Thus in a further aspect the present invention provides the use of a pharmaceutical composition comprising a polypeptide comprising
(i) the amino acid sequence of Figure 1, or
(ii) a sequence which has more than 60%, e.g. 65 or
   70%, preferably more than 80%, e.g. more than 90 or 95% homology to sequence (i) (according to the test described hereinafter) which provides a functionally equivalent protein, or
(iii) a functionally equivalent fragment of any sequence (i) or (ii); and a pharmaceutically acceptable excipient, diluent or carrier.

For the manufacture of a medicament for the prevention and/or treatment of allergic airway disease and respiratory diseases characterised by eosinophilic airway inflammation and airway hyper-responsiveness.

"Nucleic acid molecules" according to the invention may be single or double stranded DNA, cDNA or RNA, preferably DNA. Derivatives of nucleotide sequences capable of encoding functionally-equivalent polypeptides may be obtained by using conventional methods well known in the art.

Nucleic acid molecules for use in the invention may consist only of sequences derived from Figure 1 (or related functionally equivalent sequences), or may comprise additional sequences, such as structural or functional sequences, e.g. sequences which control transcription and/or expression (particularly in mammalian cells), or sequences which comprise the sequence for an additional protein moiety which may form a fusion protein which may have specific properties e.g. act as a secretary signal. Thus for example the sequence may be in the form of a vector containing the nucleic acid molecules described herein. Suitable vectors include plasmids and viruses.

"Polypeptides" as referred to herein includes both full-length protein and shorter length peptide sequences, e.g. protein fragments as described herein. Such polypeptides may be prepared by any convenient means, e.g. by isolation from the source prokaryote or by recombinant means by expression of the appropriate nucleic acid molecule in a host cell operatively linked to an expression control sequence, or a recombinant DNA cloning vehicle or vector containing such a recombinant DNA molecule or by chemical or biochemical synthesis (ex vivo).

"Sequence identity" as referred to herein in connection with nucleotide sequences refers to the value obtained when assessed using ClustalW (Thompson et al., 1994, Nucl. Acids Res., 22, p4673-4680) with the following parameters:
Pairwise alignment parameters - Method: accurate,
Matrix: IUB, Gap open penalty: 15.00, Gap extension penalty: 6.66;
Multiple alignment parameters - Matrix: IUB, Gap open penalty: 15.00, % identity for delay: 30, Negative matrix: no, Gap extension penalty: 6.66, DNA transitions weighting: 0.5.

In connection with amino acid sequences, "sequence identity" refers to sequences which have the stated value when assessed using ClustalW (Thompson et al., 1994, supra) with the following parameters: Pairwise alignment parameters - Method: accurate, Matrix: PAM, Gap open penalty: 10.00, Gap extension penalty: 0.10; Multiple alignment parameters - Matrix: PAM, Gap open penalty: 10.00, % identity for delay: 30, Penalize end gaps: on, Gap separation distance: 0, Negative matrix: no, Gap extension penalty: 0.20, Residue-specific gap penalties: on, Hydrophilic gap penalties: on, Hydrophilic residues: GPSNDQEKR. Sequence identity at a particular residue is intended to include identical residues which have simply been derivatized.

"Functionally equivalent" proteins or protein fragments refers to proteins or fragments related to, or derived from the amino acid sequence of Figure 1, where the amino acid sequence has been modified by single or multiple amino acid (e.g. at 1 to 50, e.g. 10 to 30, preferably 1 to 5 bases) substitution, addition and/or deletion but which nonetheless retains functional activity, e.g. suppresses ovalbumin-induced eosinophilia, for example reducing eosinophil numbers to the extent of more than 10 %, e.g. more than 25%, particularly preferably more than 50% and/or an increase in the production of specific cytokines such as interleukin-1β (IL-1β), IL-2, IL-6, IL-8, IL-10, IL-12, IL-12 receptor, tumour necrosis factor a (TNFα ), interferon-γ and granulocyte-macrophage-colony stimulating factor (GM-CSF) e.g. a more than 10 fold, preferably more than 100 fold increase over normal levels and/or stimulation of Thl responses.

Within the meaning of "addition" variants are included amino and/or carboxyl terminal fusion proteins or polypeptides, comprising an additional protein or polypeptide fused to the polypeptide sequence.

Particularly preferred are naturally occurring equivalents such as biological variations, e.g. allelic, geographical or allotypic variants and derivatives prepared using known techniques. For example, functionally-equivalent proteins or fragments may be prepared either by chemical peptide synthesis or in recombinant form using the known techniques of site-directed mutagenesis, random mutagenesis, or enzymatic cleavage and/or ligation of nucleic acids.

The invention is particularly directed to homologues and related molecules from different prokaryotes, e.g. from bacterial genera, species or strains, particularly from the genus *Mycobacterium, e.g.* homologues from the *Mycobacterium tuberculosis* complex which includes *M. tuberculosis, M. bovis* and M. *africanum.* Such sequences may themselves be modified, particularly derivatized providing they still retain functionality.

Derivatives of the proteins may be prepared by post-synthesis/isolation modification or by modification during synthesis, e.g. using modified residues or expression of modified nucleic acid molecules, where appropriate.

Functionally-equivalent fragments according to the invention may be made by truncation, e.g. by removal of a peptide from the N and/or C-terminal ends or by selection of an appropriate active domain region, e.g. an epitopic region which retains its functionality. Such fragments may be derived from the sequence of Figure 1 or may be derived from a functionally equivalent protein to that disclosed in Figure 1.

It will be appreciated that where functional fragments are selected they may not exhibit all functions attributed to the source molecules. Thus functionally equivalent proteins or fragments refers to retention of relevant functional properties such that the fragment retains utility according to the invention, e.g. reduces eosinophilia, increases the production of specific cytokines and/or stimulates the Thl immune response, as mentioned above. Preferably the fragments are between 6 and 99 residues in length, e.g. 15 to 99 residues, preferably 6 to 30, 10 to 25, 15 to 50 or 15 to 30 residues. Particularly preferred fragments of the sequence shown in Figure 1 are those derived from or consisting of the following residues:-
1-25
1-58
25-99
51-99
75-99

Functionally equivalent nucleic acid sequences/fragments compared to the sequence recited in Figure 1 are also used in compositions of the invention. These sequences are defined with reference to the functionally equivalent protein/peptides (as defined above) which they encode.

"Hybridisation" as used herein refers to those sequences which bind under non-stringent conditions (6 x SSC/50% formamide at room temperature) and washed under conditions of high stringency e.g. 2 x SSC, 65°C (where SSC = 0.15M NaCl, 0.015M sodium citrate, pH 7.2).

"Pharmaceutically acceptable" as referred to herein refers to ingredients that are compatible with other ingredients of the compositions as well as physiologically acceptable to the recipient.

Pharmaceutical compositions according to the invention may be formulated in conventional manner using readily available ingredients. Thus, the active ingredient (ie. the nucleic acid molecule or protein/peptide), may be incorporated, optionally together with other active substances, with one or more conventional carriers, diluents and/or excipients, to produce conventional galenic preparations such as tablets, pills, powders, lozenges, sachets, cachets, elixirs, suspensions, emulsions, solutions, syrups, aerosols (as a solid or in a liquid medium), ointments, soft and hard gelatin capsules, suppositories, sterile injectable solutions, sterile packaged powders, and the like.

As mentioned above, compositions may additionally comprise molecules which assist or augment the action of the nucleic acid molecules or polypeptides described hereinbefore, e.g. thalidomide (and analogues thereof), low dose cyclophosphamide, LPS, cytokines, chemokines, CpG oligodeoxynucleotides and other immunomodulators and/or antiinflammatory agents such as cytokine antagonists or glucocorticosteroids.

Thus for example, the compositions may be used together with active ingredients for specific immunotherapies e.g. in cancer vaccines. Appropriate immunotherapy treatment/vaccine preparations which may include nucleic acid molecules/polypeptides as described herein include subunit vaccines or treatments based on tumour specific antigens or tumour associated antigens or antibody, anti-idiotype antibody or whole cell preparations for vaccination or therapy. When used in therapy or vaccination the nucleic acid molecules or polypeptides described herein may provide (or encode) an antigen resulting in a specific immune response directed to that antigen and/or may result in a general and nonspecific immune response. In the latter case in which compositions containing other active ingredients are used, the nucleic acid molecules/polypeptides described herein act as adjuvants and may be used for this purpose.

Preventative or therapeutic preparations may be formulated to include one or more suitable adjuvants, e.g. Incomplete Freund's Adjuvant, BCG, Montanide, aluminium hydroxide, saponin, quil A, or more purified forms thereof, muramyl dipeptide, mineral or vegetable oils, Novasome or non-ionic block co-polymers or DEAE dextran, in the presence of one or more pharmaceutically acceptable carriers or diluents. Suitable carriers include liquid media such as saline solution.

Examples of suitable carriers, excipients, and diluents are lactose, dextrose, sucrose, sorbitol, mannitol, starches, gum acacia, calcium phosphate, aglinates, tragacanth, gelatin, calcium silicate, microcrystalline cellulose, polyvinylpyrrolidone, cellulose, water syrup, water, water/ethanol, water/glycol, water/polyethylene glycol, propylene glycol, methyl cellulose, methylhydroxybenzoates, propyl hydroxybenzoates, talc, magnesium stearate, mineral oil or fatty substances such as hard fat or suitable mixtures thereof. The compositions may additionally include lubricating agents, wetting agents, emulsifying agents, suspending agents, preserving agents, sweetening agents, flavouring agents, and the like. The compositions of the invention may be formulated so as to provide quick, sustained or delayed release of the active ingredient after administration to the patient by employing procedures well known in the art.

Compositions may be in an appropriate dosage form, for example as an emulsion or in liposomes, niosomes, microspheres, nanoparticles or the like.

If required, the compositions may also contain targeting moieties attached to the active ingredient, e.g. a ligand which binds specifically and selectively to an endogenous receptor to allow targeting to a particular cell type or location, such as targeting to lymphocytes, monocytes, macrophages, endothelial cells, epithelial cells, blood cells, erythrocytes, platelets, eosinophils, neutrophils, natural killer cells, dendritic cells, brain cells, heart cells, lung cells, islet cells, kidney cells, cancer cells, hormonal gland cells, skin, bone, joints, bone marrow, gastric mucosa, lymph nodes, Peyers patches, the omentum and other immunological tissues.

The above described compositions have utility in the treatment or prophylaxis of cancer, allergic reactions and/or conditions typified by a Th2-type immune response and/or conditions associated with eosinophilia.

Alternatively viewed, the present invention provides a use for treating or preventing allergic airway disease and respiratory diseases characterised by eosinophilic airway inflammation and airway hyper-responsiveness, said patient is to be administered a pharmaceutical composition as described hereinbefore.

Furthermore, the present invention provides the use of a nucleic acid molecule comprising
(i) the nucleotide sequence of Figure 1, or
(ii) a sequence which has more than 66%, e.g. 70 or 75%, preferably more than 80%, e.g. more than 90 or 95% identity to sequence (i) (according to the test described hereinbefore) or a sequence which hybridizes to sequence (i) under conditions of 2 x SSC, 65°C (wherein SCC = 0.15M NaCl, 0.015M sodium citrate, pH 7.2) which encodes a functionally equivalent protein to the sequence encoded by the nucleotide sequence of Figure 1, or (iii) a fragment of sequence (i) or (ii) encoding a functionally equivalent protein fragment; or
a polypeptide comprising
(i) the amino acid sequence of Figure 1, or
(ii) a sequence which has more than 60%, e.g. 65 or 70%, preferably more than 80%, e.g. more than 90 or 95% homology to sequence (i) (according to the test described hereinbefore) which provides a functionally equivalent protein, or
(iii) a functionally equivalent fragment of sequence (i) or (ii);
in the preparation of a medicament for treating or preventing allergic airway disease and respiratory diseases characterised by eosinophilic airway inflammation and airway hyper-responsiveness.

As defined herein "treatment" refers to reducing, alleviating or eliminating one or more symptoms of the condition which is being treated, relative to the symptoms prior to treatment. For example, symptoms which may be affected include eosinophilia, decreased secretion of particular cytokines, a Th2-biased immune response, tumour size (e.g. by halting proliferation, causing differentiation, enhancing or inducing antitumour immune responses or causing some cell death), allergic response, presence of autoantibodies, etc which are treated to achieve the effects particularly as defined in respect of the functional properties of functionally equivalent polypeptides.

"Prevention" of a condition refers to delaying or preventing the onset of a condition or reducing its severity, as assessed by the appearance or extent of one or more symptoms of said condition.

Cancers which may be prevented or treated include malignant and pre-malignant or benign tumours and include carcinomas, sarcomas, glioma, melanoma and Hodgkin's disease, including cancers of the bladder, kidney, pancreas, brain, head and neck, breast, gut, prostate, lung and ovary and leukaemias and lymphomas.

Allergic conditions which may be treated or prevented include eczema, dermatitis, allergic rhinitis, allergic airway diseases, hyper-eosinophilic syndrome, and respiratory diseases characterized by eosinophilic airway inflammation and airway hyperresponsiveness, such as allergic asthma, intrinsic asthma, allergic bronchopulmonary aspergillosis, eosinophilic pneumonia, allergic bronchitis bronchiectasis, occupational asthma, reactive airway disease syndrome, interstitial lung disease, hypereosinophilic syndrome.

Preferably however the composition is used for treating asthma. In a further preferred feature, the composition is used for treating respiratory diseases in which eosinophilia plays a role, e.g. allergies (as described above, particularly asthma) and pulmonary eosinophilia.

Patients which may be treated include, but are not limited to mammals, particularly primates, domestic animals and livestock. Thus preferred animals for treatment include mice, rats, guinea pigs, cats, dogs, pigs, goats, sheep, horses and particularly preferably, humans.

As mentioned previously, either nucleic acid molecules or polypeptides may be used according to the invention. In instances in which nucleic acid molecules are employed, these are conveniently applied in a form to allow their expression within the patient, thus providing a form of gene therapy. Thus the pharmaceutical compositions described herein containing a nucleic acid molecule may be used in methods of gene therapy.

Thus for example the nucleic acid molecules may be provided in a liposome, micelle or other convenient carrying vehicle which may comprise targeting moieties to allow its targeting to cells of interest.

Alternatively the molecules may be packaged in other, "vehicles" such as viruses, plasmids or cells (particularly transfected species-matched cells) which are all well known in the art for this purpose which allow expression of the resident molecule.

Appropriate techniques for transfection are well known and include electroporation, microinjection, lipofection, adsorption, viral transfection and protoplast fusion.

Administration of compositions of the invention may take place by any of the conventional routes, e.g. by inhalation, nasally, orally, rectally or parenterally, such as by intramuscular, subcutaneous, intraperitoneal or intravenous injection. Treatment or prophylaxis by topical application of a composition, e.g. an ointment, to the skin is also possible. Optionally administration may be performed at intervals, e.g. 2 or more applications, e.g. 2-4 applications at hourly, daily, weekly or monthly intervals, e.g. several times a day, or every 3-5 days, or at fortnightly, monthly or quarterly intervals.

It has been observed in work conducted on the related molecule cpn 60.2 that the route of administration may affect the immune response which is generated. For example when Mtcpn 60.2 is administered intranasally, a Th2 to Th1 shift is stimulated although the reverse effect is observed when administered intraperitoneally. Thus, the route of administration should take into account the disorder to be treated/prevented and thus for example in treating autoimmune disorders, intraperitoneal administration may be appropriate whereas treatment or prevention of particularly allergic disorders may be for example by intranasal administration.

In prophylactic methods of the invention, administration (conveniently orally or by inhalation or subcutaneous or intramuscular injection) is preferably performed at more lengthy intervals, e.g. intervals of 2-12 weeks. For therapeutic purposes, administration (conveniently orally or by inhalation or intravenous injection) is performed 1-4 times in a single day or over 2 days.

The active ingredient in composition of the invention may comprise from about 0.01% to about 99% by weight of the formulation, preferably from about 0.1 to about 50%, for example 10%. The compositions are preferably formulated in a unit dosage form, e.g. with each dosage containing from about 0.01mg to about 1g of the active ingredient, e.g. 0.05mg to 0.5g, for a human, e.g. 1-100mg.

The precise dosage of the active compound to be administered and the length of the course of treatment will, of course, depend on a number of factors including for example, the age and weight of the patient, the specific condition requiring treatment and its severity, and the route of administration. Generally however, an effective dose may lie in the range of from about 0.1µg/kg to about 14mg/kg, preferably 0.1 to 1mg/kg, e.g. from about 1mg to 1g of polypeptide per day, depending on the animal to be treated and the dosage form, taken as a single dose. Thus for example, an appropriate daily dose for an adult may be from 7µg to 1g, e.g. 10mg to 1g per day, e.g. 25 to 500mg of the polypeptide per day.

Similar or lower dosages may be used when using nucleic acid molecules described herein, e.g. from about 0.2ng/kg to about 2.5mg/kg (e.g. from about 0.2ng/kg to about 2ng/kg or about 1.5ng/kg to about 2.5mg/kg) such as about 14ng to about 175mg for an adult. However, where the nucleic acid molecules are packaged in cells or vectors proportionally higher or lower amounts may be required depending on the extent of non-cpn encoding DNA and sequences which influence the level of expression, e.g. 5 or 10-fold larger amounts, e.g. nucleic acid molecules described herein packaged in a vector may be used at about 1.0ng/kg to about 12.5mg/kg.

As mentioned above, the family of polypeptides defined herein and the nucleic acid molecules encoding them stimulate the production of a set of cytokines. This therefore allows the use of these compounds for the express purpose of stimulating production of these cytokines whether or not this occurs in a therapeutic/prophylactic situation. Thus in a further aspect the present invention provides an in vitro method of stimulating cytokine production in a cell, derived from a patient having an allergic airway disease and/or a respiratory disease characterised by eosinophilic airway inflammation and airway hyper-responsiveness wherein
a nucleic acid molecule comprising
(i) the nucleotide sequence of Figure 1, or
(ii) a sequence which has more than 66%, e.g. 70 or 75%, preferably more than 80%, e.g. more than 90 or 95% identity to sequence (i) (according to the test described hereinbefore) or a sequence which hybridizes to sequence (i) under conditions of 2 x SSC, 65°C (wherein SCC = 0.15M NaCl, 0.015M sodium citrate, pH 7.2) which encodes a functionally equivalent protein to the sequence encoded by the nucleotide sequence of Figure 1, or (iii) a fragment of sequence (i) or (ii) encoding a functionally equivalent protein fragment; or
a polypeptide comprising
(i) the amino acid sequence of Figure 1, or
(ii) a sequence which has more than 60%, e.g. 65 or 70%, preferably more than 80%, e.g. more than 90 or 95% homology to sequence (i) (according to the test described hereinbefore) which provides a functionally equivalent protein, or
(iii) a functionally equivalent fragment of sequence (i) or (ii); to said cell, are to be administered.

Such methods may be performed *in vitro,* e.g. on cells, tissues or organs outside the body. This methodology may for example be used in research methods to identify the molecule or molecules which react or bind to or are activated via molecules of the invention, e.g. cpn 10 receptor molecules As a corollary to such methods, the stimulation of cytokine production may be used to measure the presence of molecules of the invention.

Thus, in a further aspect the present invention provides an in vitro a method of assessing the presence or concentration of a polypeptide or peptide of the invention in a sample wherein said sample is applied to a cell derided from a patient having an allergic airway disease and/or a respiratory disease characterised by eosinophilic airway inflammation and airway hyper-responsiveness and the level of production of one or more cytokines is measured and compared to the level of production of said one or more cytokines in a control sample wherein the increase over control levels provides a correlation to the presence or concentration of said polypeptide or peptide in said sample.

As used herein "control" refers to a sample which does not contain molecules of the invention or moieties which increase production of the cytokine(s) to be measured. Where appropriate, standard curves may be generated using molecules of the invention to allow quantitative assessment to be made of the presence or concentration of said molecules, although qualitative assessments may also be made. This method may furthermore be used to identify molecules of the invention.

This may have beneficial therapeutic or prophylactic effects and in which case the invention extends to the nucleic acid molecules and polypeptides as described above for use in treating conditions which may be alleviated, overcome or prevented by increasing specific cytokines, and the use of such molecules for the preparation of medicaments for that purpose.

Preferably the cytokines which are increased, e.g. more than 10 or 100 fold relative to normal levels, are selected from the group consisting of IL-1β, IL-2, IL-6, IL-8, IL-10, IL-12, TNFα, interferon-γ and GM-CSF.

### Definitions

"AUTOIMMUNE DISEASE". This term intended to cover those cases where it can be shown that the autoimmune process contributes to the pathogenesis of a disease. Such diseases are typically associated with a Thelper lymphocyte-1 (Th-1) type immune response.
"ALLERGIC CONDITIONS". This term is intended to cover conditions asociated with a T helper lymphocyte-2 (Th-2) type immune response. In allergic reaction, high IgE levels occur and Th-2 immune responses predominate over Th-1 responses, resulting in inflammatory response. Examples of allergic conditions include the following: asthma, rhinitis/hay fever, eczema and anaphylaxis.
"ADJUVANT". This term is intended to cover any substance which, when incorporated into or administered simultaneously with antigen, potentiates the immune response.
"Mtcpn10", "cpn 10", "hsp10", and "Pep10" are used interchangeably throughout the specification to refer to the amino acid sequence shown in Figure 1.

The invention will now be described in more detail by way of the following non-limiting Examples in which:-
Figure 1 shows the nucleotide and amino acid sequence of cpn 10 from *M tuberculosis;*
Figure 2 shows the reduction in eosinophil levels in mice with ovalbumin-induced pulmonary eosinophilia after the administration of 5 doses of Mtcpn 10 ("Pep10").
Figures 3 and 4 show the levels of IL4 (Fig 3) and INF-γ (Fig 4)detected in BALs.

### Materials

*Expression and purification of chaperonin 60 proteins M. tuberculosis* cpn 10 was prepared by Prof M. Singh (WHO Collaborating Centre, Germany) using conventional chromatography as described below.

### Purification of recombinant cpn 10:

The 10kDa antigen was expressed in a recombinant *E. coli* strain (IPTG-induced) as a fusion protein with maltose binding protein (MBP) using the commercially available pMAL-c vector (New England Biolabs). Initial purification was performed on an amylose affinity column. Afterwards the fusion protein was cleaved with factor Xₐ and the 10kDa antigen was further purified by anion exchange chromatography, dialysed against 10mM ammonium bicarbonate, aliquotted and lyophilized.

Great care was taken to check each batch of protein for LPS contamination using the Limulus assay (Tabona et al., 1998, J. Immunol., 161, p1414-1421). If LPS contamination was detected it was removed on a polymyxin B affinity column and levels of LPS re-assayed. Recombinant, LPS-low, chaperonins were further purified on a Reactive Red column to remove contaminating proteins and peptides (Tabona et al., 1998, supra).

The *in vitro* effects of cpn 10 on the production of IL-1β, IL-6, IL-8, IL-10, IL-12, TNFα and GM-CSF in human PBMCs was determined using 2-site ELISA as described by Tabona et al., 1998, supra.

### EXAMPLE 1: Mycobacterium tuberculosis cpn 10 suppresses asthma in the mouse

This Example shows for the first time that in a murine model of allergic inflammation *M*. *tuberculosis* cpn 10 protein inhibited the recruitment of eosinophils to the airways in immunized mice. These data show that Mtcpn 10 modulates airway inflammation in the mouse and therefore, has important implications for allergic disease treatment and prevention.

### Methods

*Murine Model of Inflammation* - A murine model of allergic inflammation that allows the quantitation of eosinophil and T lymphocyte recruitment in the airways following antigen challenge has been developed. Furthermore, a state of the art pulmonary monitoring system is used which allows changes in pulmonary mechanics to bronchoconstrictor agonists *in vivo* to be determined.

*Immunisation Protocol -* C57B1/6 wild type (local supplier) 6 - 8 weeks old mice were immunised with ovalbumin (10 µg intraperitoneal injection; in 1 mg aluminium hydroxide) on day 0 and repeated 7 days later. On days 14, 15 and 16 mice were placed in a plexiglass container (12 L) and exposed to a nebulised solution of ovalbumin (10 mg/ml; De Vilibiss Ultraneb 90). Sham immunised wild type mice were injected with (1 mg aluminium hydroxide) on days 0 and 7 and also challenged with ovalbumin on days 14-16. Aerosol exposure was performed by exposure 3 times daily for 20 min at hourly intervals and bronchoalveolar lavage, collection of lungs for immunohistochemistry and lung mechanics was performed 24 h after the last aerosol challenge.

While ovalbumin is not a respiratory allergen often encountered by asthmatic subjects, the Th2 responses observed in murine models of inflammation are analogous to those observed following immunization with house dust mite. The Th2 cytokine profile generated by both allergens are similar. The advantage of using ovalbumin is that it is easily available and the specific activity of this allergen does not change between batches and therefore, we can control for antigen dose between batches.

*BCG treatment:* Six days following immunization with ovalbumin (10 µg), mice were injected with BCG (log BCG viable units: -4, -5 and -6) via the intravenous route. On day 7, mice received a booster injection with ovalbumin (10µg). On day 13, mice received a second administration of BCG at the same dose and route as described for day 6. On day 14, mice were placed in a plexiglass box and exposed three times with nebulized ovalbumin (1%solution) for a period of 30 minutes at 1 hour intervals. This procedure was repeated on day 15 and 16. 24 hours after the last ovalbumin exposure, mice were anaesthetised and a bronchoalveolar lavage performed for the enumeration of eosinophils.

*cpn 10 treatment:* Mice from the same batch of animals were immunized to ovalbumin and treated with Mtcpn 10 (10 µg/animal) by direct instillation into the trachea. Mice were treated with Mtcpn 10 on day 6 and day 13 and then 30 min before the commencement of the challenge protocol on days 14, 15, and 16 (a total of 5 treatments).

### Results

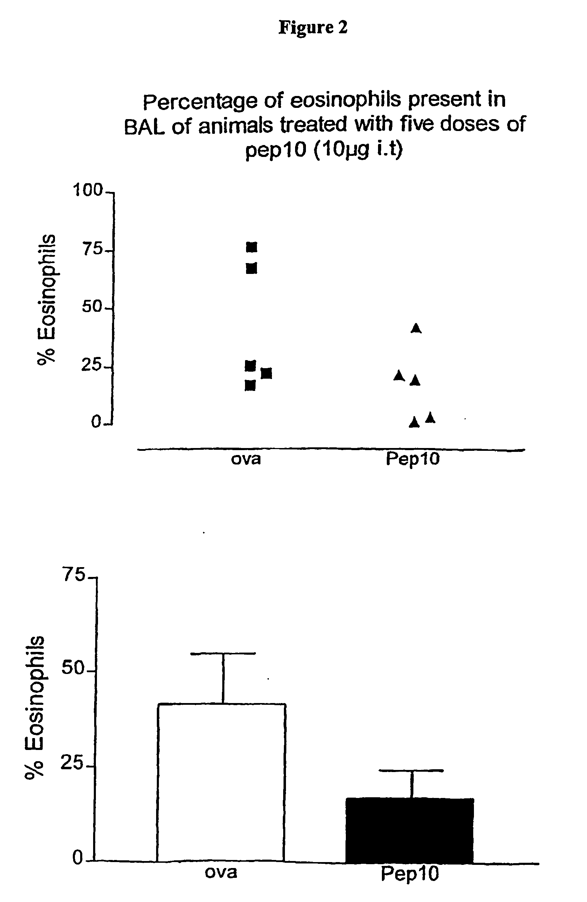

The results are shown in Figure 2. There was significant suppression of the recruitment of eosinophils to the airways following ovalbumin challenge suggesting a protective effect for Mtcpn 10 ("Pep10") in asthma.

### Cytokine measurements

We investigated the effect of cpn 10 over cytokine production in the serum and lavage collected 24 hours after the last challenge. We performed measurements of IL-4, IL-5 and INF-γ levels in samples collected from groups of mice treated with 10µgof cpn 10 and 56µg of 60.1, both administered in a 5 doses scheme of treatment.

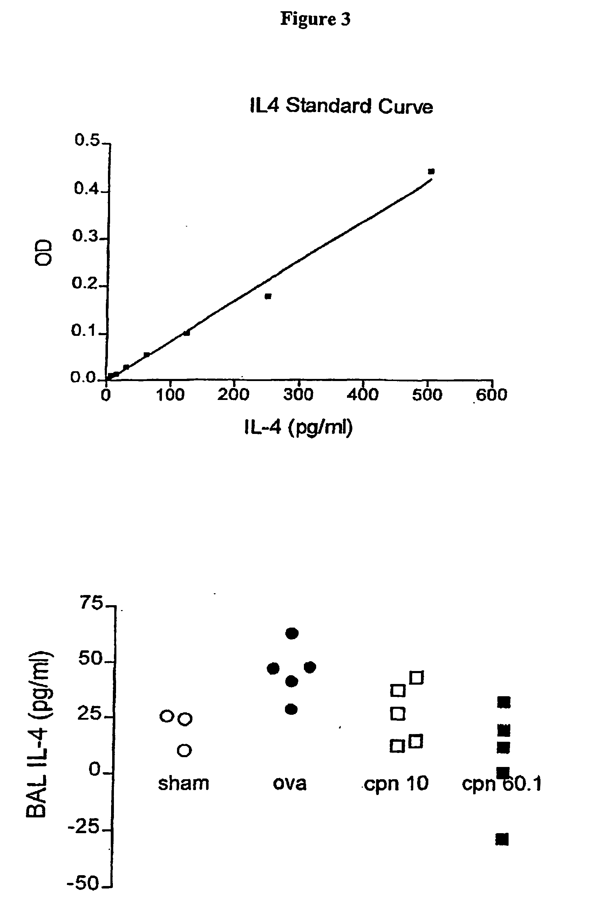

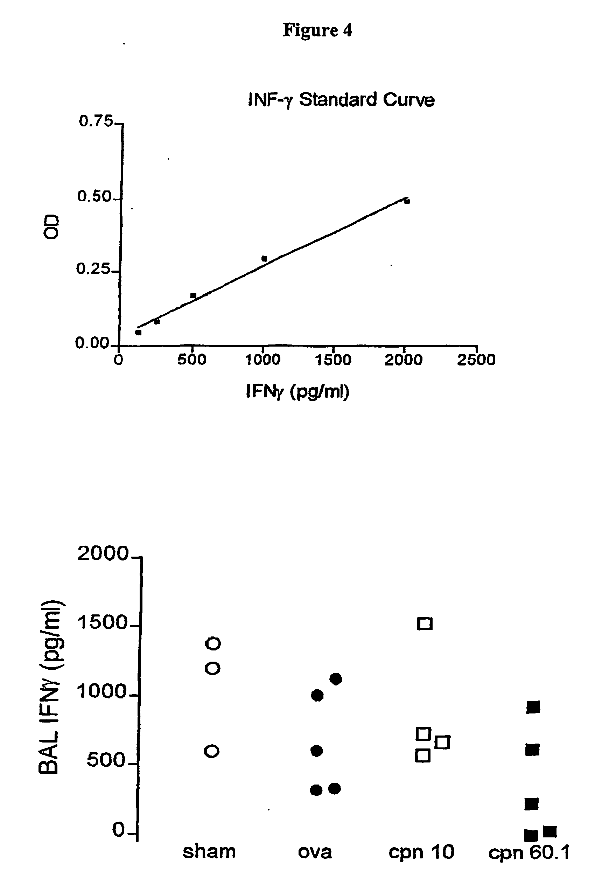

It was not possible to detect cytokine levels in the serum 24 hours after the last challenge and IL5 was not detected in any sample at this time. The levels of IL4 and INF-γ detected in BALs are shown in **Fig 3** and **Fig 4.**

An important advantage of cpn 10 is that it can provide a prophylactic agent as distinct from an agent which is used to treat acute symptoms. In other words, cpn10 treatment can prevent allergic conditions such as asthma from developing.

The use of cpn 10 in the prevention and treatment of asthma is described in this example. Skilled persons will understand that the results obtained are relevant to other allergic conditions such as rhinitis/hay fever, eczema and anaphylaxis because all of the allergic conditions have a common mechanism (over-reactivity of Th-2 cells). Hence, if cpn10 can inhibit asthma, it should also inibit the other allergic conditions.

These data demonstrate for the first time that Mtcpn 10 can suppress eosinophilic inflammation in a murine model of asthma. This show that this protein has the potential to modulate airways inflammation in the mouse, which has important implications for the treatment and prevention of allergic airway disease, and respiratory diseases characterised by eosinophilic airway inflammation and airway hyper-responsiveness.

## Claims

1. The use of a pharmaceutical composition in the manufacture of a medicament for the prevention and/or treatment of allergic airway disease and respiratory diseases **characterised by** eosinophilic airway inflammation and airway hyper-responsiveness wherein the pharmaceutical composition comprises a nucleic acid molecule comprising
(i) the nucleotide sequence of Figure 1, or
(ii) a sequence which has more than 66%, e.g. 70 or 75%, preferably more than 80%, e.g. more than 90 or 95% identity to sequence (i) or a sequence which hybridizes to sequence (i) under conditions of 2 x SSC, 65°C (wherein SCC = 0.15M NaCl, 0.015M sodium citrate, pH 7.2) which encodes a functionally equivalent protein to the sequence encoded by the nucleotide sequence of Figure 1, or
(iii) a fragment of sequence (i) or (ii) encoding a functionally equivalent protein fragment; and a pharmaceutically acceptable excipient, diluent or carrier.

2. The use of a pharmaceutical composition in the manufacture of a medicament for the prevention and/or treatment of allergic airway disease and respiratory diseases **characterised by** eosinophilic airway inflammation and airway hyper-responsiveness wherein the pharmaceutical composition comprises a polypeptide comprising
(i) the amino acid sequence of Figure 1, or
(ii) a sequence which has more than 60%, e.g. 65 or 70%, preferably more than 80%, e.g. more than 90 or 95% homology to sequence (i) which provides a functionally equivalent protein, or
(iii) a functionally equivalent fragment of any sequence (i) or (ii); and a pharmaceutically acceptable excipient, diluent or carrier.

3. The use as claimed in Claim 2 wherein the fragments are between 6 and 99 residues in length.

4. The use as claimed in Claim 3 wherein the fragment lengths are between 6 to 60.

5. The use as claimed in Claim 3 wherein the fragments are derived from or consisting of at least one of the following residues of the sequence shown in Figure 1:
1-25
1-58
25-99
51-99
75-99.

6. The use as claimed in any previous claim wherein the respiratory disease treated is selected from at least one of the following conditions: allergic rhinitis, allergic asthma, intrinsic asthma, allergic bronchopulmonary aspergillosis, eosinophilic pneumonia, allergic bronchitis bronchiectasis, occupational asthma, reactive airway disease syndrome, interstitial lung disease or hypereosinophilic syndrome.

7. The use as claimed in Claim 6 wherein the respiratory disease is asthma.

8. The use as claimed in Claim 6 wherein the respiratory disease is allergic rhinitis.

9. The use of a pharmaceutical composition as defined in any one of Claims 1 to 8 in the manufacture of a medicament for the treatment and/or prevention allergic airway disease and respiratory diseases **characterized by** eosinophilic airway inflammation and airway hyper-responsiveness. wherein the medicament comprises a therapeutically or prophylactically effective dose, or plurality of doses, of the composition.

10. An in vitro method of stimulating cytokine production in a cell derived from a patient having an allergic airway disease and/or a respiratory disease **characterised by** eosinophilic airway inflammation and airway hyper-responsiveness wherein said method comprises administration of a composition comprising a nucleic acid molecule comprising
(i) the nucleotide sequence of Figure 1, or
(ii) a sequence which has more than 66%, e.g. 70 or 75%, preferably more than 80%, e.g. more than 90 or 95% identity to sequence (i) or a sequence which hybridizes to sequence (i) under conditions of 2 x SSC, 65°C (wherein SCC =0.15M NaCl, 0.015M sodium citrate, pH 7.2) which encodes a functionally equivalent protein to the sequence encoded by the nucleotide sequence of Figure 1, or
(iii) a fragment of sequence (i) or (ii) encoding a functionally equivalent protein fragment; or
a polypeptide comprising
(i) the amino acid sequence of Figure 1, or
(ii) a sequence which has more than 60%, e.g. 65 or 70%, preferably more than 80%, e.g. more than 90 or 95% homology to sequence (i) which provides a functionally equivalent protein, or
(iii) a functionally equivalent fragment of sequence (i) or (ii); to said cell; and a pharmaceutically acceptable excipient, diluent or carrier.

11. A method as claimed in Claim 10 wherein the cytokine production is increased at least 10-fold relative to normal levels.

12. A method as claimed in Claim 10 or 11 wherein the cytokines are selected from at least one of the group consisting of IL-1 β, IL-2, IL-6, IL-8, IL-10, IL-12, TNFα, interferon γ and GM-CSF.

13. The use of a pharmaceutical composition in the manufacture of a medicament for treatment and/or prevention of allergic airway disease and respiratory diseases **characterised by** eosinophilic airway inflammation and airway hyper-responsiveness by stimulation of cytokine production in a diseased cell, wherein said composition comprises a nucleic acid molecule comprising
(i) the nucleotide sequence of Figure 1, or
(ii) a sequence which has more than 66%, e.g. 70 or 75%, preferably more than 80%, e.g. more than 90 or 95% identity to sequence (i) or a sequence which hybridises to sequence (i) under conditions of 2 x SSC, 65°C (wherein SCC = 0.15M NaCl, 0.015M sodium citrate, pH 7.2) which encodes a functionally equivalent protein to the sequence encoded by the nucleotide sequence of Figure 1, or
(iii) a fragment of sequence (i) or (ii) encoding a functionally equivalent protein fragment; or
a polypeptide comprising
(i) the amino acid sequence of Figure 1, or
(ii) a sequence which has more than 60%, e.g. 65 or 70%, preferably more than 80%, e.g. more than 90 or 95% homology to sequence (i) which provides a functionally equivalent protein, or
(iii) a functionally equivalent fragment of sequence (i) or (ii),
and a pharmaceutically acceptable excipient, diluent or carrier.

14. A use as claimed in Claim 13 wherein the cytokine production is increased at least 10-fold relative to normal levels.

15. A use as claimed in Claim 13 or 14 wherein the cytokines are selected from at least one of the group consisting of IL-1β, IL-2, IL-6, IL-8, EL-10, IL-12, TNFα, interferon γ and GM-CSF.

16. An in vitro method of assessing the presence or concentration of a polypeptide or peptide as defined in any preceding claim in a sample wherein said sample is applied to a cell derived from a patient having an allergic airway disease and/or a respiratory disease **characterised by** eosinophilic airway inflammation and airway hyper-responsiveness and the level of production of one or more cytokines is measured and compared to the level of production of said one or more cytokines in a control sample wherein the increase over control levels provides a correlation to the presence or concentration of said polypeptide or peptide in said sample.

## Patentansprüche

1. Verwendung einer pharmazeutischen Zusammensetzung bei der Herstellung eines Medikamentes für die Prävention und/oder Behandlung von allergischen Luftwegs- und Atmungsorgan-Erkrankungen, die durch eine eosinophile Luftwegs-Entzündung und eine Luftwegs-Überempfindlichkeit **gekennzeichnet** sind, wobei die pharmazeutische Zusammensetzung ein Nukleinsäure-Molekül, das folgendes umfasst:
i) die Nukleotidsequenz aus Fig. 1 oder
ii) eine Sequenz, die zu mehr als 66 %, beispielsweise 70 oder 75 %, vorzugsweise zu mehr als 80 %, beispielsweise mehr als 90 oder 95 % mit der Sequenz (i) identisch ist, oder eine Sequenz, die unter den Bedingungen von 2 x SSc 65°C (wobei SCC = 0,15M NaCl, 0,015M Natriumzitrat bei einem pH-Wert von 7,2 bedeutet) zur Sequenz (i) hybridisiert und ein Protein kodiert, das zu der Sequenz funktional äquivalent ist, die durch die Nukleotidsequenz aus Fig. 1 kodiert wird, oder
iii) ein Fragment der Sequenz (i) oder (ii), das ein funktional äquivalentes Proteinfragment kodiert,
und einen pharmazeutisch akzeptablen Zusatz, Verdünner oder Träger umfasst.

2. Verwendung einer pharmazeutischen Zusammensetzung bei der Herstellung eines Medikamentes für die Prävention und/oder Behandlung von allergischen Luftwegs- und Atmungsorgan-Erkrankungen, die durch eine eosinophile Luftwegs-Entzündung und eine Luftwegs-Überempfindlichkeit charakterisiert sind, wobei die pharmazeutische Zusammensetzung ein Polypeptid, das folgendes umfasst:
i) die Aminosäuresequenz aus Fig. 1, oder
ii) eine Sequenz, die zu mehr als 60 %, beispielsweise 65 oder 70 %, vorzugsweise zu mehr als 80 % , beispielsweise 90 oder 95 % zur Sequenz (i) homolog ist und ein funktional äquivalentes Protein liefert, oder
iii) ein funktional äquivalentes Fragment einer der Sequenzen (i) oder (ii),
und einen pharmazeutisch akzeptablen Zusatzstoff, Verdünner oder Träger umfasst.

3. Verwendung nach Anspruch 2, wobei die Fragmente eine Länge zwischen 6 und 99 Residuen aufweisen.

4. Verwendung nach Anspruch 3 wobei das Fragment eine Länge von 6 bis 60 Residuen aufweist.

5. Verwendung nach Anspruch 3 wobei die Fragmente aus wenigstens einem der folgenden Residuen der in Fig. 1 dargestellten Sequenz bestehen oder hiervon abgeleitet sind:
1 - 25
1 - 58
25 - 99
51 - 99
75 - 99.

6. Verwendung nach einem der vorhergehenden Ansprüche, wobei die zu behandelnde Atmungsorgan-Erkrankung wenigstens einer der folgenden Krankheitszustände ist: allergische Rhinitis, allergisches Asthma, nicht allergisches Asthma, allergische bronchopulmonale Aspergillose, eosinophile Pneumonie, allergische Bronchitis-Bronchiektase, berufsbedingtes Asthma, reaktives Luftweg-Erkrankungssyndrom, interstitielle Lungenveränderungen oder hyper-eosinophiles Syndrom.

7. Verwendung nach Anspruch 6, wobei die Atmungsorgan-Erkrankung Asthma ist.

8. Verwendung nach Anspruch 6, wobei die Atmungsorgan-Erkrankung eine allergische Rhinitis ist.

9. Verwendung einer pharmazeutischen Zusammensetzung nach einem der Ansprüche 1 bis 8 bei der Herstellung eines Medikaments für die Behandlung und/oder Prävention von allergischen Luftwegserkrankungen und Atmungsorgan-Erkrankungen, die durch eine eosinophile Luftwegsentzündung und eine Luftwegs-Überempfindlichkeit **gekennzeichnet** sind, wobei das Medikament eine therapeutisch oder prophylaktisch wirksame Dosis oder Vielzahl von Dosen der Zusammensetzung enthält.

10. In vitro durchgeführtes Verfahren zur Stimulierung der Zytokin-Produktion in einer Zelle, die von einem Patienten erhalten ist, der eine allergische Luftwegserkrankung und/oder eine Atmungsorgan-Erkrankung aufweist, die durch eine eosinophile Luftwegsentzündung und Luftwegs-Überempfindlichkeit **gekennzeichnet** ist, wobei dieses Verfahren die Verabreichung einer Zusammensetzung umfasst, die ein Nukleinsäuremolekül, das aus folgendem besteht:
i) der Nukleotidsequenz aus Fig. 1 oder
ii) einer Sequenz, die zu mehr als 66 %, beispielsweise 70 oder 75 %, vorzugsweise zu mehr als 80 %, beispielsweise mehr als 90 oder 95 % mit der Sequenz (i) identisch ist, oder eine Sequenz, die unter den Bedingungen von 2 x SSc 65°C (wobei SCC = 0,15M NaCl, 0,015M Natriumzitrat bei einem pH-Wert von 7,2 bedeutet) zur Sequenz (i) hybridisiert und ein Protein kodiert, das zu der Sequenz funktional äquivalent ist, die durch die Nukleotidsequenz aus Fig. 1 kodiert wird, oder
iii) einem Fragment der Sequenz (i) oder (ii), das ein funktional äquivalentes Proteinfragment kodiert; oder
ein Polypeptid, das aus folgendem besteht:
i) der Aminosäuresequenz aus Fig. 1, oder
ii) einer Sequenz, die zu mehr als 60 %, beispielsweise 65 oder 70 %, vorzugsweise zu mehr als 80 % , beispielsweise 90 oder 95 % zur Sequenz (i) homolog ist und ein funktional äquivalentes Protein liefert, oder
iii) einem funktional äquivalentes Fragment einer der Sequenzen (i) oder (ii), für diese Zelle
und einen pharmazeutisch akzeptablen Zusatzstoff, Verdünner oder Träger umfasst.

11. Verfahren nach Anspruch 10, bei dem die Zytokin-Produktion bezogen auf normale Werte um das wenigstens zehnfache erhöht ist.

12. Verfahren nach Anspruch 10 oder 11, wobei die Zytokine aus wenigstens einem Element der folgenden Gruppe ausgewählt sind: IL-1β, IL-2, IL-6, IL-8, IL-10, IL-12, TNFα, Interferon γ und GM-CSF.

13. Verwendung einer pharmazeutischen Zusammensetzung bei der Herstellung eines Medikamentes zur Behandlung und/oder Prävention einer allergischen Luftwegserkrankung und von Atmungsorgan-Erkrankungen, die durch eine eosinophile Luftwegsentzündung und Luftwegs-Überempfindlichkeit **gekennzeichnet sind, durch** Stimulation der Zytokin-Produktion in einer erkrankten Zelle, wobei diese Zusammensetzung ein Nukleinsäuremolekül, das folgendes umfasst:
i) die Nukleotidsequenz aus Fig. 1 oder
ii) eine Sequenz, die mehr zu als 66 %, beispielsweise 70 oder 75 %, vorzugsweise zu mehr als 80 %, beispielsweise mehr als 90 oder 95 % mit der Sequenz (i) identisch ist, oder eine Sequenz, die unter den Bedingungen von 2 x SSc 65°C (wobei SCC = 0,15M NaCl, 0,015M Natriumzitrat bei einem pH-Wert von 7,2 bedeutet) zur Sequenz (i) hybridisiert und ein Protein kodiert, das zu der Sequenz funktional äquivalent ist, die **durch** die Nukleotidsequenz aus Fig. 1 kodiert wird, oder
iii) ein Fragment der Sequenz (i) oder (ii), das ein funktional äquivalentes Proteinfragment kodiert; oder
ein Polypeptid, das folgendes umfasst:
i) die Aminosäuresequenz aus Fig. 1, oder
ii) eine Sequenz, die zu mehr als 60 %, d. h. 65 oder 70 %, vorzugsweise zu mehr als 80 % , d. h. 90 oder 95 % zur Sequenz (i) homolog ist und ein funktional äquivalentes Protein liefert, oder
iii) ein funktional äquivalentes Fragment einer der Sequenzen (i) oder (ii),
und einen pharmazeutisch akzeptablen Zusatzstoff, Verdünner oder Träger umfasst.

14. Verfahren nach Anspruch 13, bei dem die Zytokin-Produktion bezogen auf normale Werte um das wenigstens zehnfache erhöht ist.

15. Verwendung nach Anspruch 13 oder 14, wobei die Zytokine aus wenigstens einem Element der folgenden Gruppe ausgewählt sind: IL-1β, IL-2, IL-6, IL-8, IL-10, IL-12, TNFα, Interferon γ und GM-CSF.

16. In vitro durchgeführtes Verfahren zur Bestätigung des Vorhandenseins oder der Konzentration eines Polypeptids oder Peptids nach einem der vorhergehenden Ansprüche in einer Probe, wobei diese Probe auf eine Zelle angewendet wird, die von einem Patienten erhalten wurde, der eine allergische Luftwegserkrankung und/oder eine Luftwegserkrankung aufweist, die durch eine eosinophile Luftwegsentzündung und eine Luftwegs-Überempfindlichkeit **gekennzeichnet** ist, wobei das Ausmaß der Produktion von einem oder mehreren Zytokinen gemessen und mit dem Ausmaß der Produktion dieses einen oder dieser mehreren Zytokine in einer Kontrollprobe verglichen wird, wobei die Erhöhung über die Kontrollpegel eine Korrelation zum Vorhandensein oder zur Konzentration des Polypeptids oder Peptids in der Probe liefert.

## Revendications

1. Utilisation d'une composition pharmaceutique dans la fabrication d'un médicament pour la prévention et/ou le traitement d'une maladie allergique des voies aériennes et de maladies respiratoires **caractérisées par** une inflammation éosinophile des voies aériennes et une hyper-réactivité des voies aériennes, dans laquelle la composition pharmaceutique comprend une molécule d'acide nucléique comprenant
(i) la séquence de nucléotides de la figure 1, ou
(ii) une séquence qui présente une identité avec la séquence (i) supérieure à 66%, par exemple de 70% ou de 75%, de préférence supérieure à 80%, par exemple supérieure à 90% ou à 95%, ou une séquence qui s'hybride à la séquence (i) dans des conditions de 2 x SSC, à 65°C (où SCC = NaCl 0,15 M, citrate de sodium 0,015 M, pH 7,2) qui code pour une protéine fonctionnellement équivalente à la séquence codée par la séquence de nucléotides de la figure 1, ou
(iii) un fragment de la séquence (i) ou (ii) codant pour un fragment de protéine fonctionnellement équivalente ; et un excipient, un diluant ou un véhicule acceptable sur le plan pharmaceutique.

2. Utilisation d'une composition pharmaceutique dans la fabrication d'un médicament pour la prévention et/ou le traitement d'une maladie allergique des voies aériennes et de maladies respiratoires **caractérisées par** une inflammation éosinophile des voies aériennes et une hyper-réactivité des voies aériennes, dans laquelle la composition pharmaceutique comprend un polypeptide comprenant
(i) la séquence d'acides aminés de la figure 1, ou
(ii) une séquence qui présente une homologie avec la séquence (i) supérieure à 60%, par exemple de 65% ou de 70%, de préférence supérieure à 80%, par exemple supérieure à 90% ou à 95% qui fournit une protéine fonctionnellement équivalente, ou
(iii) un fragment de l'une quelconque des séquences (i) ou (ii) fonctionnellement équivalent ; et un excipient, un diluant ou un véhicule acceptable sur le plan pharmaceutique.

3. Utilisation selon la revendication 2, dans laquelle les fragments ont une longueur comprise entre 6 et 99 résidus.

4. Utilisation selon la revendication 3, dans laquelle les longueurs des fragments sont comprises entre 6 et 60.

5. Utilisation selon la revendication 3, dans laquelle les fragments sont dérivés ou constitués d'au moins un des résidus suivants de la séquence illustrée sur la figure 1 :
1 - 25
1 - 58
25 - 99
51 - 99
75 - 99.

6. Utilisation selon l'une quelconque des revendications précédentes, dans laquelle la maladie respiratoire traitée est choisie parmi au moins une des maladies suivantes : la rhinite allergique, l'asthme allergique, l'asthme intrinsèque, l'aspergillose broncho-pulmonaire allergique, la pneumonie à éosinophiles, la bronchiectasie, la bronchite allergique, l'asthme professionnel, le syndrome d'irritation bronchique, la pneumopathie interstitielle ou le syndrome d'hyperéosinophilie.

7. Utilisation selon la revendication 6, dans laquelle la maladie respiratoire est l'asthme.

8. Utilisation selon la revendication 6, dans laquelle la maladie respiratoire est la rhinite allergique.

9. Utilisation d'une composition pharmaceutique selon l'une quelconque des revendications 1 à 8 dans la fabrication d'un médicament pour le traitement et/ou la prévention d'une maladie allergique des voies aériennes et de maladies respiratoires **caractérisées par** une inflammation éosinophile des voies aériennes et une hyper-réactivité des voies aériennes, dans laquelle le médicament comprend une dose, ou une pluralité de doses, de la composition efficaces sur le plan thérapeutique ou prophylactique.

10. Procédé de stimulation *in vitro* de la production de cytokines dans une cellule dérivée d'un patient présentant une maladie allergique des voies aériennes et/ou une maladie respiratoire **caractérisée par** une inflammation éosinophile des voies respiratoires et une hyper-réactivité des voies respiratoires, dans lequel ledit procédé comprend l'administration à ladite cellule d'une composition comprenant :
une molécule d'acide nucléique comprenant :
(i) la séquence de nucléotides de la figure 1, ou
(ii) une séquence qui présente une identité avec la séquence (i) supérieure à 66%, par exemple de 70% ou de 75%, de préférence supérieure à 80%, par exemple supérieure à 90% ou à 95%, ou une séquence qui s'hybride à la séquence (i) dans des conditions de 2 x SSC, à 65°C (où SCC = NaCl 0,15 M, citrate de sodium 0,015 M, pH 7,2) qui code pour une protéine fonctionnellement équivalente à la séquence codée par la séquence de nucléotides de la figure 1, ou
(iii) un fragment de la séquence (i) ou (ii) codant pour un fragment de protéine fonctionnellement équivalente ; ou
un polypeptide comprenant :
(i) la séquence d'acides aminés de la figure 1, ou
(ii) une séquence qui présente une homologie avec la séquence (i) supérieure à 60%, par exemple de 65% ou de 70%, de préférence supérieure à 80%, par exemple supérieure à 90% ou à 95% qui fournit une protéine fonctionnellement équivalente, ou
(iii) un fragment de la séquence (i) ou (ii) fonctionnellement équivalent ;
et un excipient, un diluant ou un véhicule acceptable sur le plan pharmaceutique.

11. Procédé selon la revendication 10, dans lequel la production de cytokines est augmentée d'au moins 10 fois par rapport aux quantités normales.

12. Procédé selon la revendication 10 ou 11, dans lequel les cytokines sont choisies parmi au moins une cytokine du groupe constitué par IL-1β, IL-2, IL-6, IL-8, IL-10, IL-12, TNFα, l'interféron γ et GM-CSF.

13. Utilisation d'une composition pharmaceutique dans la fabrication d'un médicament pour la prévention et/ou le traitement d'une maladie allergique des voies aériennes et de maladies respiratoires **caractérisées par** une inflammation éosinophile des voies aériennes et une hyper-réactivité des voies aériennes par la stimulation de la production de cytokines dans une cellule malade, dans laquelle ladite composition pharmaceutique comprend :
une molécule d'acide nucléique comprenant :
(i) la séquence de nucléotides de la figure 1, ou
(ii) une séquence qui présente une identité avec la séquence (i) supérieure à 66%, par exemple de 70% ou de 75%, de préférence supérieure à 80%, par exemple supérieure à 90% ou à 95%, ou une séquence qui s'hybride à la séquence (i) dans des conditions de 2 x SSC, à 65°C (où SCC = NaCl 0,15 M, citrate de sodium 0,015 M, pH 7,2) qui code pour une protéine fonctionnellement équivalente à la séquence codée par la séquence de nucléotides de la figure 1, ou
(iii) un fragment de la séquence (i) ou (ii) codant pour un fragment de protéine fonctionnellement équivalente ; ou
un polypeptide comprenant :
(i) la séquence d'acides aminés de la figure 1, ou
(ii) une séquence qui présente une homologie supérieure à 60%, par exemple de 65% ou de 70%, de préférence supérieure à 80%, par exemple supérieure à 90% ou à 95% avec la séquence (i) qui fournit une protéine fonctionnellement équivalente, ou
(iii) un fragment de la séquence (i) ou (ii) fonctionnellement équivalent ;
et un excipient, un diluant ou un véhicule acceptable sur le plan pharmaceutique.

14. Utilisation selon la revendication 13, dans laquelle la production de cytokines est augmentée d'au moins 10 fois par rapport aux quantités normales.

15. Utilisation selon la revendication 13 ou 14, dans laquelle les cytokines sont choisies parmi au moins une cytokine du groupe constitué par IL-1β, IL-2, IL-6, IL-8, IL-10, IL-12, TNFα, l'interféron γ et GM-CSF.

16. Procédé d'évaluation *in vitro* de la présence ou de la concentration, dans un échantillon, d'un polypeptide ou d'un peptide tel que défini dans l'une quelconque des revendications précédentes, dans lequel ledit échantillon est appliqué à une cellule dérivée d'un patient présentant une maladie allergique des voies aériennes et/ou une maladie respiratoire **caractérisée par** une inflammation éosinophile des voies aériennes et une hyper-réactivité des voies aériennes, et dans lequel le niveau de production d'une ou de plusieurs cytokines est mesuré et comparé au niveau de production desdites une ou plusieurs cytokines d'un échantillon témoin dans lequel l'augmentation par rapport aux niveaux témoins fournit une corrélation avec la présence ou la concentration dudit polypeptide ou peptide dans ledit échantillon.
